(19) Europäisches Patentamt
European Patent Office
Office européen des brevets

(11) Publication number: 0 102 213
A1

(12) # EUROPEAN PATENT APPLICATION

(21) Application number: 83304667.5

(22) Date of filing: 12.08.83

(51) Int. Cl.³: C 07 D 295/08
A 61 K 31/495

(30) Priority: 21.08.82 GB 8224111
30.03.83 GB 8308846

(43) Date of publication of application:
07.03.84 Bulletin 84/10

(84) Designated Contracting States:
CH DE FR GB IT LI NL

(71) Applicant: BEECHAM GROUP PLC
Beecham House Great West Road
Brentford Middlesex(GB)

(72) Inventor: Ainsworth, Anthony Trevor
7 Redcroft Walk
Cranleigh Surrey(GB)

(72) Inventor: Smith, David Glynn
36 Shrewsbury Road
Redhill Surrey(GB)

(74) Representative: Russell, Brian John et al,
European Patent Attorney Beecham Pharmaceuticals
Great Burgh Yew Tree Bottom Road
Epsom Surrey, KT18 5XQ(GB)

(54) **Ethanolamine derivatives, pharmaceutical compositions containing them, and processes for preparing them.**

(57) Compounds of formula (I):

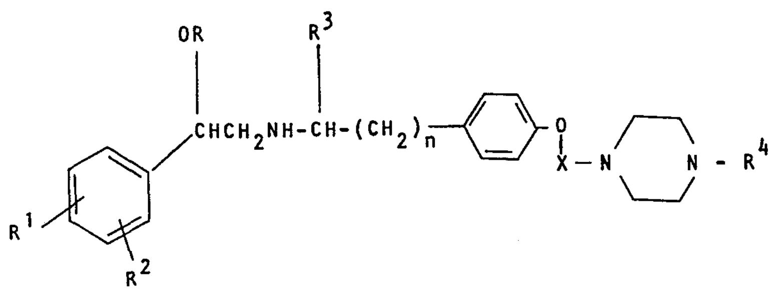

(I)

or salts thereof,
wherein
R is hydrogen or $C_{1-6}$ alkyl,
$R^1$ is hydrogen, or fluorine,
$R^2$ is hydrogen, halogen or trifluoromethyl,
$R^4$ is hydrogen, $C_{1-6}$ alkyl, optionally substituted benzyl, or optionally substituted phenyl,
X is $C_{2-10}$ straight or branched alkylene having at least two carbon atoms interposed between the oxygen and nitrogen atoms.
and n is 1 or 2,
have been found to possess anti-obesity and or anti-hyperglycaemic activity.

EP 0 102 213 A1

ETHANOLAMINE DERIVATIVES, PHARMACEUTICAL COMPOSITIONS CONTAINING THEM, AND PROCESSES FOR PREPARING THEM.

The present invention relates to derivatives of ethanolamine which have anti-obesity and/or anti-hyperglycaemic activity, to processes for their production and to their use in medicine. This activity is coupled with low cardiac-stimulant activity for particular compounds of the invention.

According to the present invention there is provided a compound of formula (I):

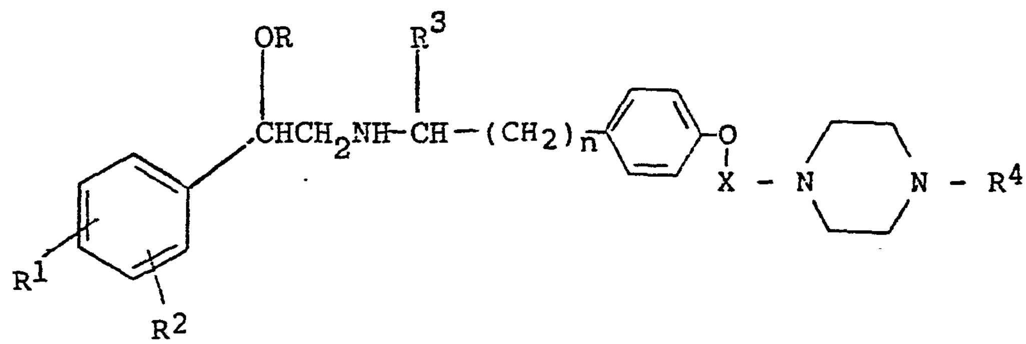

(I)

or a salt thereof,

wherein R is hydrogen or $C_{1-6}$ alkyl,

$R^1$ is hydrogen, or fluorine,

$R^2$ is hydrogen, halogen or trifluoromethyl,

$R^3$ is hydrogen or methyl,

$R^4$ is hydrogen, $C_{1-6}$ alkyl, optionally substituted benzyl, or optionally substituted phenyl,

X is $C_{2-10}$ straight or branched alkylene having at least two carbon atoms interposed between the oxygen and nitrogen atoms

and n is 1 or 2

Preferably, R is hydrogen or methyl. Suitably $R^1$ is hydrogen. Preferably $R^2$ is a meta-substituent and is hydrogen, trifluoromethyl or chlorine. X is preferably ethylene, and $R^3$ is preferably methyl.

Suitable substituents for the phenyl and benzyl groups, $R^4$, include halogen, hydroxyl, $C_{1-4}$ alkyl, $C_{1-4}$ alkoxyl, amino, mono- and di-($C_{1-4}$alkyl)amino, nitro, cyano, carboxyl and trifluoromethyl substituents.

The salts of compounds of formula (I) are preferably, but not essentially, pharmaceutically

acceptable as they are also useful in the preparation of other compounds of formula (I) and in the separation of stereoisomers of compounds of formula (I) when the counter ion is also optically active.

Pharmaceutically acceptable salts of compounds of formula (I) include acid addition salts formed with a pharmaceutically acceptable acid such as hydrochloric acid, hydrobromic acid, orthophosphoric acid, sulphuric acid, methane sulphonic acid, toluenesulphonic acid, acetic acid, propionic acid, lactic acid, citric acid, fumaric acid, malic acid, succinic acid, salicylic acid or acetylsalicylic acid.

When $R^3$ is a methyl group the compounds of formula (I) have two asymmetric carbon atoms, ie the carbon atom bearing the phenyl and -OR groups and the carbon atom bearing $R^3$. Such compounds may, therefore, exist in four stereoisomeric forms. The present invention encompasses all stereoisomers of the compounds of formula (I) whether free or admixed with other stereoisomers in any proportion and thus includes, for instance, racemic mixtures of enantiomers.

Preferably the carbon atom bearing the phenyl and -OR groups has the R-absolute configuration.

Most preferably, both the carbon atom bearing the phenyl and -OR groups and that bearing $R^3$, when $R^3$ is a methyl group, have the R-absolute configuration, since such compounds are generally the most potent.

The absolute configuration of any compound of formula (I) may be determined by conventional X-ray crystallographic techniques.

The present invention also provides a process for producing a compound of formula (I), or a salt thereof, which process comprises reducing a keto-group and/or double bond and/or cleaving a benzyl group(s) of a compound of formula (II):

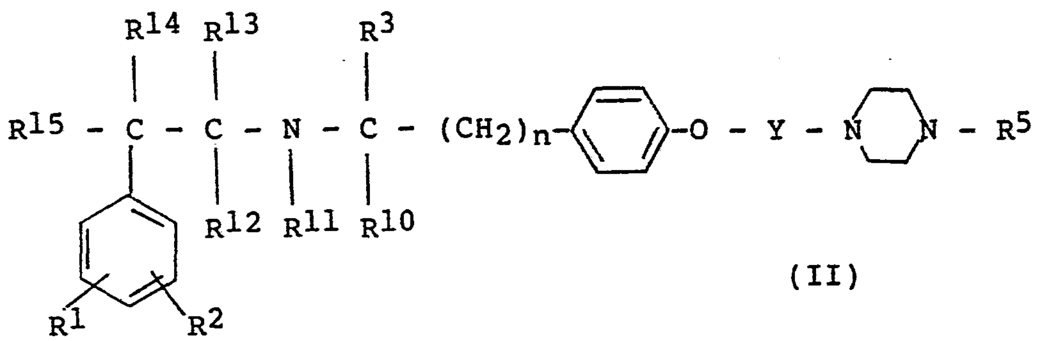

(II)

wherein $R^1$, $R^2$, $R^3$, and n are as defined in relation to formula (I);

$R^5$ is a group $R^4$ as defined in relation to formula (I) or a group reducible to the group $R^4$;

$R^{10}$ is hydrogen or together with $R^{11}$ forms a bond;

$R^{11}$ is hydrogen or benzyl or together with $R^{10}$ or $R^{12}$ forms a bond;

$R^{12}$ is hydrogen or together with $R^{11}$ forms a bond or together with $R^{13}$ forms an oxo-group;

$R^{13}$ is hydrogen or together with $R^{12}$ forms an oxo-group;

$R^{14}$ is hydrogen or together with $R^{15}$ forms an oxo-group;

$R^{15}$ is hydroxyl or $C_{1-6}$ alkoxy, or together with $R^{14}$ forms an oxo-group;

Y is a group X as defined in relation to formula (I) or a group reducible to a group X,

provided that there is no more than one oxo-group represented by any of $R^{12}$ to $R^{15}$;

and optionally thereafter forming a salt of the compound of formula (I) so formed, and/or converting the compound of formula (I) so formed into a further compound of formula (I).

Where there are two or more reducible functional groups in the compound of formula (II) these may be reduced separately in any order or simultaneously.

The aforementioned reductions may be effected by conventional chemical methods or by catalytic methods. Suitably, chemical reduction may be effected with lithium aluminium hydride, sodium cyanoborohydride, sodium borohydride or borane methyl sulphide. Catalytic hydrogenation may be carried out using catalysts such as palladium on charcoal, or platinum, for instance, as reduced platinum oxide.

Reduction by sodium borohydride is conveniently effected in a lower alkanolic solvent such as methanol or ethanol. The reaction is generally carried out at from 0-20° C.

Reduction by lithium aluminium hydride is conveniently effected in a dry, ether solvent such as diethyl ether or tetrahydrofuran at ambient or elevated temperatures.

Catalytic reduction is conveniently effected in a conventional hydrogenation solvent such as a lower alkanol, for instance ethanol. The hydrogenation is generally carried out under hydrogen gas at about 1 to 10 atmospheres pressure and at ambient or elevated temperatures.

Reduction of a compound of formula (II) wherein $R^5$ or $R^{11}$ is benzyl is conveniently effected by catalytic

hydrogenation, preferably using palladium on charcoal as catalyst.

Preferred aspects of the process of the invention comprise reducing a compound of formula (IIA):

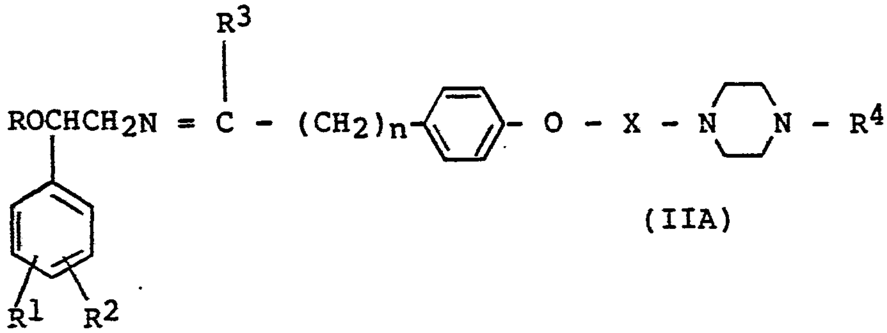

(IIA)

or reducing a compound of formula (IIB):

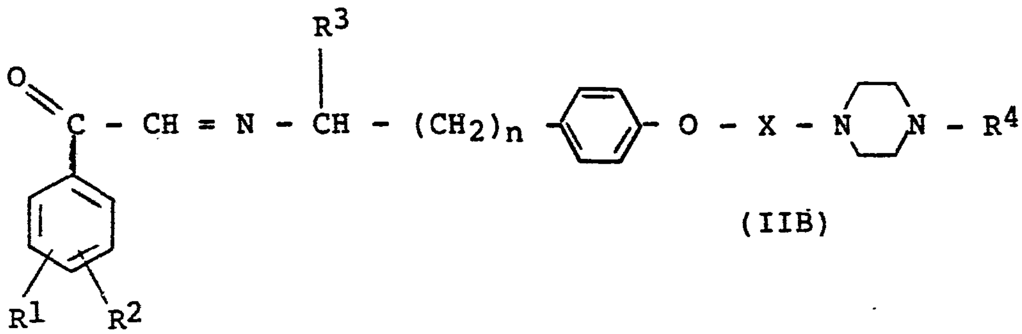

(IIB)

or reducing a compound of formula (IIC):

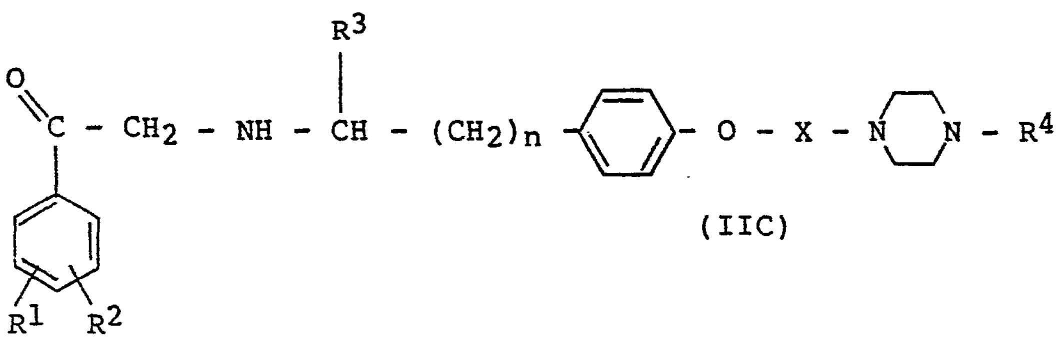

(IIC)

or reducing a compound of formula (IID):

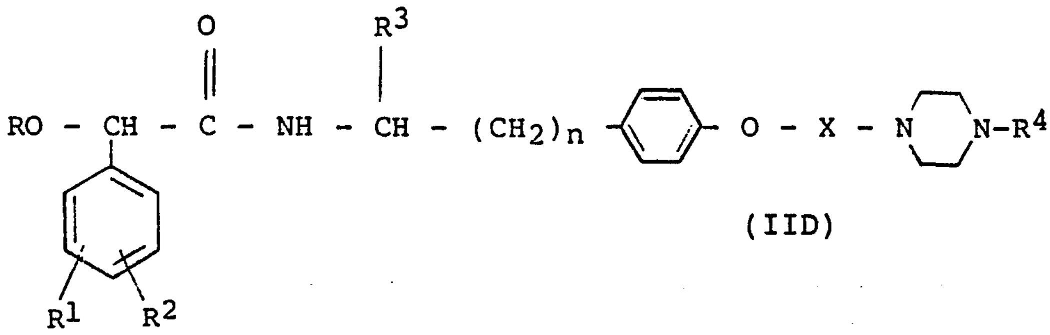

(IID)

or reducing a compound of formula (IIE):

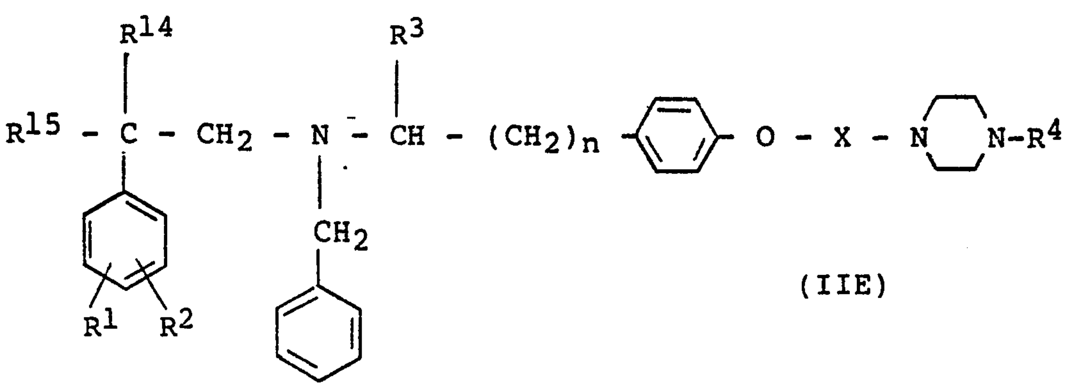

(IIE)

or reducing a compound of formula (IIF):

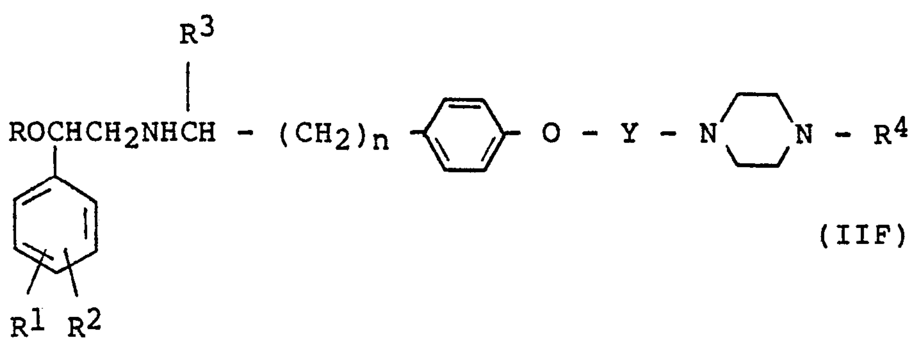

(IIF)

or reducing a compound of formula (IIG):

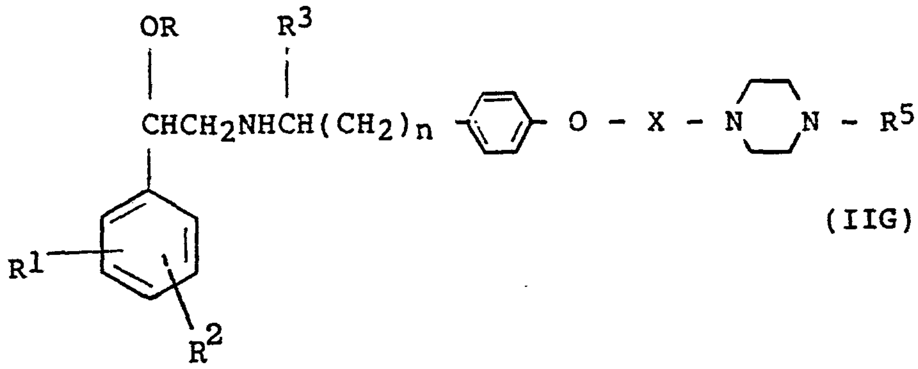

(IIG)

wherein R, $R^1$, $R^2$, $R^3$, $R^4$, X and n are as defined in relation to formula (I), and $R^5$, $R^{14}$, $R^{15}$ and Y are as defined in relation to formula (II).

The present invention also provides another process for producing a compound of formula (I) or a salt thereof, which process comprises reacting a compound of formula (III):

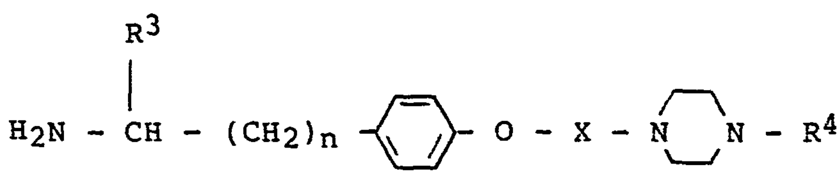

(III)

wherein $R^3$, $R^4$, X and n are as defined in relation to formula (I);

with a compound of formula (IV):

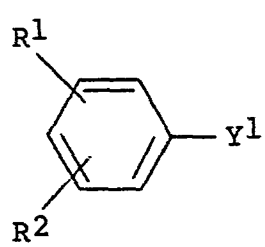

(IV)

wherein $R^1$ and $R^2$ are as defined in relation to formula (I),

and $Y^1$ is a group capable of reacting with the primary amino group of a compound of formula (III), thus forming a compound of formula (I) directly, and optionally thereafter forming a salt of the compound of formula (I) so formed and/or converting the compound of formula (I) so formed into a further compound of formula (I).

Typical examples of compounds of formula (IV) are compounds of formulae (IVA) and (IVB):

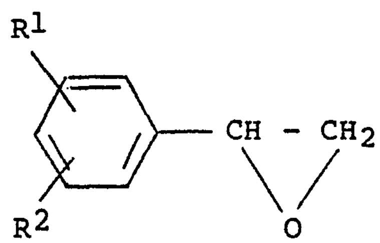

(IVA)

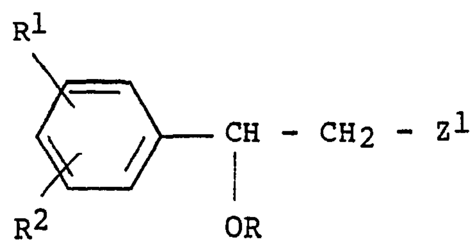

(IVB)

wherein R, $R^1$ and $R^2$ are as defined in relation to formula (I)

and $Z^1$ is a leaving group, preferably halogen or a tosyloxy group.

The reaction of a compound of formula (III) with a compound of formula (IVA) is conveniently effected in a solvent such as a lower alkanol, preferably ethanol.

The reaction of a compound of formula (III) with a compound of formula (IVB) is conveniently conducted in a solvent, such as dimethyl sulphoxide, at elevated temperature, preferably about 50°C.

The present invention provides a further process for the production of compounds of formula (I) or salts thereof, which process comprises reacting a compound of formula (V):

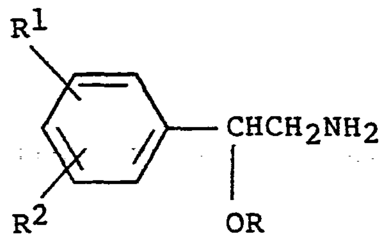

(V)

wherein R, $R^1$ and $R^2$ are as defined in relation to formula (I);

with a compound of formula (VI):

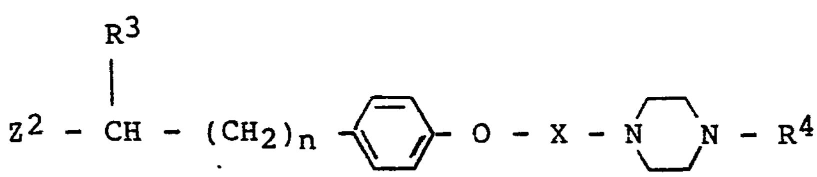

(VI)

wherein $R^3$, $R^4$, X and n are as defined in relation to formula (I)

and $Z^2$ is a leaving group preferably halogen or tosyloxy.

The reaction of a compound of formula (V) with a compound of formula (VI) is conveniently effected in a solvent such as dimethylsulphoxide at an elevated temperature, preferably about 50°C for about two or three days.

A preferred process for producing compounds of formula (I) comprises the reduction of a compound of formula (IIA), especially using sodium borohydride in methanol at ambient temperatures.

The salts of compounds of formula (I) may be produced by treating the compound of formula (I) with the appropriate acid.

Compounds of formula (I) and salts thereof, produced by the above processes, may be recovered from the reaction medium and purified by conventional methods.

Compounds of formula (II) may be produced by reacting a compound of formula (III) as hereinbefore defined with a compound of formula (VII):

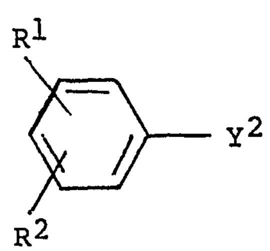

(VII)

wherein $R^1$ and $R^2$ are as defined in relation to formula (I)

and $Y^2$ is a group which is capable of reacting with the amine of formula (III) thus forming a compound of formula (II).

Typical examples of compounds of formula (VII) are:

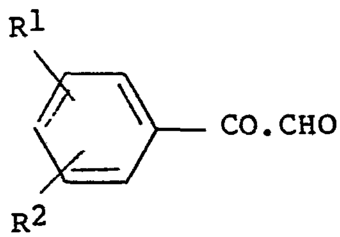

(VIIA)

or its hydrate or hemi-acetal of a lower alkanol;

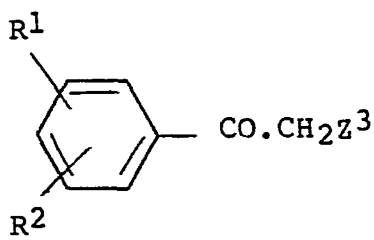

(VIIB)

wherein $Z^3$ is a leaving group, preferably bromine;

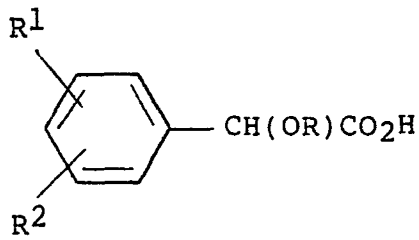

(VIIC)

wherein R, $R^1$ and $R^2$ are as defined in relation to formula (I).

Conventional conditions compatible with the particular compound of formula (VII) may be used for this reaction. Thus, the reaction of a compound of formula (VIIA) with a compound of formula (III) is conveniently conducted at elevated temperatures under conditions resulting in the removal of the water formed during the reaction. A particularly suitable method is to perform the reaction in a solvent, such as benzene, under reflux and to remove the water azeotropically using a Dean and Stark trap.

The reaction of a compound of formula (VIIB) with a compound of formula (III) is conveniently conducted in a polar organic solvent such as acetonitrile or butanone, at an elevated temperature, for instance under reflux.

The reaction of a compound of formula (VIIC) with a compound of formula (III) is conveniently conducted under standard peptide formation reaction conditions.

Alternatively a compound of formula (II) may be prepared by reacting a compound of formula (VIII):

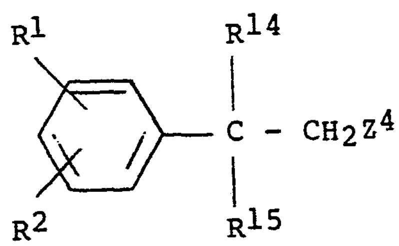

(VIII)

wherein $R^1$, $R^2$, $R^{14}$ and $R^{15}$ are as defined in relation to formula (II);

and $Z^4$ is a leaving group, preferably halogen or tosyloxy;

with a compound of formula (IX):

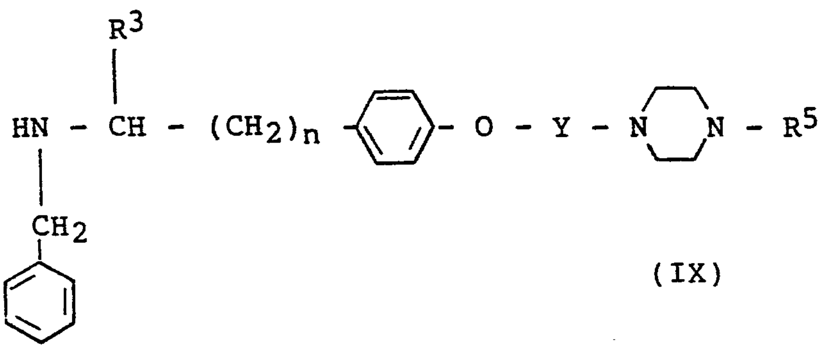

(IX)

wherein $R^3$, and n are as defined in relation to formula (I), and Y and $R^5$ are as defined in relation to formula (II).

A particularly preferred process for producing certain compounds of formula (II) comprises reacting a compound of formula (V) as hereinbefore defined with a compound of the formula (X):

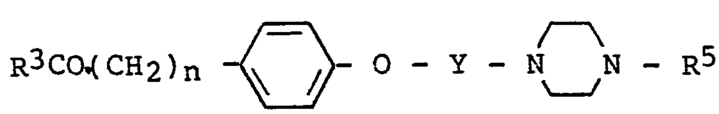

(X)

wherein $R^3$ and n are as defined in relation to formula (I), and Y and $R^5$ are as defined in relation to formula (II).

The reaction of a compound of formula (V) with a compound of formula (X) is conveniently effected under conditions which result in the removal of water formed during the reaction. A particularly suitable method is to perform the reaction in a solvent, such as benzene, under reflux and to remove the water azeotropically using a Dean and Stark trap.

It is often convenient to prepare the compound of formula (II) and reduce it, in situ, to the desired compound of formula (I) without isolation of the compound of formula (II).

Those compounds of formula (I) having more than one asymmetric carbon atom may be separated into diastereoisomeric pairs of enantiomers by, for example, fractional crystallisation from a suitable solvent such as ethyl acetate. The pair of enantiomers thus obtained may be separated into individual stereoisomers by conventional means such as by the use of an optically active acid as a resolving agent.

Suitable optically active acids which may be used as resolving agents are described in 'Topics in Stereochemistry', Vol. 6, Wiley Interscience, 1971, Allinger, N.L., and Eliel, W.L. Eds.

Alternatively any enantiomer of a compound of formula (I) may be obtained by stereospecific synthesis using optically pure starting materials of known configuration.

By reacting a single enantiomer of a compound of formula (III) with a single enantiomer of a compound of formula (IVA) or (IVB), the direct stereospecific synthesis of a single enantiomer of a compound of formula (I) is effected. Thus, for example, reaction of a compound of formula (IVA) with the R-absolute configuration with a single enantiomer of a compound of formula (III) in which the carbon atom bearing $R^3$, when $R^3$ is a methyl group, has the R-absolute configuration would afford a compound of formula (I) with the R,R-absolute configuration.

By using single enantiomers of a compound of formula (III) and of a compound of formula (VIIC) a stereospecific synthesis of a compound of formula (II) is achieved. This may then be reduced to a compound of formula (I) without altering the configuration of the asymmetric carbon atoms. Thus, for example, reaction of a compound of formula (VIIC) with the R-absolute configuration and a single enantiomer of a compound of formula (III), in which the carbon atom bearing $R^3$, when $R^3$ is a methyl group, has the R-absolute configuration, would afford a compound of formula (II) and, on reduction, a single enantiomer of the compound of formula (I) with the R,R-absolute configuration.

A compound of formula (I) or a pharmaceutically acceptable salt thereof (hereinafter 'the drug') may be administered as the pure drug, however, it is preferred that the drug be administered as a pharmaceutical composition also comprising a pharmaceutically acceptable carrier.

Accordingly, the present invention also provides a pharmaceutical composition comprising a compound of formula (I) or a pharmaceutically acceptable salt thereof with a pharmaceutically acceptable carrier therefor.

As used herein the terms 'pharmaceutical composition' and 'pharmaceutically acceptable' embrace compositions and ingredients for both human and veterinary use.

Usually the compositions of the present invention will be adapted for oral administration although compositions for administration by other routes, such as by injection are also envisaged.

Particularly suitable compositions for oral administration are unit dosage forms such as tablets and capsules. Other fixed unit dosage forms, such as powders presented in sachets, may also be used.

In accordance with conventional pharmaceutical practice the carrier may comprise a diluent, filler, disintegrant, wetting agent, lubricant, colourant, flavourant, or the like.

Typical carriers may, therefore, comprise such agents as microcrystalline cellulose, starch, sodium starch glycollate, polyvinylpyrrolidone, polyvinyl-polypyrrolidone, magnesium stearate, sodium lauryl sulphate, sucrose, and the like.

Most suitably the composition will be provided in unit dose form. Such unit doses will normally comprise

0.1 to 500 mg of the drug, more usually 0.1 to 250 mg and favourably 0.1 to 100 mg.

The present invention further provides a method for treating obesity in human or non-human animals, which method comprises administering an effective, non-toxic amount of a compound of formula (I) or a pharmaceutically acceptable salt thereof to obese humans or non-human animals.

The present invention further provides a method for treating hyperglycaemia in humans or non-human animals which method comprises administering an effective, non-toxic amount of a compound of formula (I) or a pharmaceutically acceptable salt thereof to hyperglycaemic humans or non-human animals.

Conveniently, the drug may be administered as a pharmaceutical composition as hereinbefore defined, and this forms a particular aspect of the present invention.

In treating hyperglycaemic or obese humans the drug may be taken in doses, such as those described above, one to six times a day in a manner such that the total daily dose for a 70 kg adult will generally be about 0.1 to 1000 mg, and more usually about 1 to 500 mg.

In treating hyperglycaemic or obese animals, especially dogs, the drug may be administered by mouth, usually once or twice a day and at about 0.025 mg/kg to 10 mg/kg, for example 0.1 mg/kg to 2 mg/kg.

The invention will now be illustrated with reference to the following Examples.

As used in the Examples, the term 'diastereoisomer' refers to a pair of enantiomers.

In the following examples all temperatures are in $^{o}C$, all pressures are in mm.Hg.

EXAMPLE 1.

N-[2-(4-(2-(1-(4-Benzyl)piperazinyl)ethoxy)phenyl)-1-methylethyl]-2-hydroxy-2-(3-trifluoromethylphenyl)ethanamine trihydrochloride

A mixture of 2-hydroxy-2-(3-trifluoromethylphenyl)ethanamine (3.36g) and 1-[4-(2-(1-(4-benzyl)piperazinyl)ethoxy)phenyl]propan-2-one (5.97g) in benzene was treated under reflux using a Dean and Stark apparatus until the theoretical amount of water had been evolved. The solution was cooled, the solvent removed and the residue dissolved in methanol. Sodium borohydride (4.5g) was added in portions and the solution allowed to stir at room temperature for 0.5h. The solvent was evaporated, the residue partitioned between water and ethyl acetate and the organic layer separated and dried. Filtration and evaporation yielded an oil which was chromatographed on Kieselgel 60. Elution with methanol-chloroform (4:96) gave an oil which was dissolved in ether and treated with ethereal hydrogen chloride to give N-[2-(4-(2-(1-(4-Benzyl)piperazinyl)ethoxy)phenyl)-1-methylethyl]-2-hydroxy-2-(3-trifluoromethylphenyl)ethanamine trihydrochloride, m.p. 190-196°(methanol-ethyl acetate) as a 55:45 mixture of diastereoisomers.

$^1$H-n.m.r. ;-

δ (DMSO-$d_6$)

1.15 (3H,d), 2.5-3.9 (15H,m, + 1H,v.broad,disappears with $D_2O$); 4.4 (4H,m); 5.25 (1H,m), 6.95 (2H,d), 7.25 (2H,d), 7.35-7.9 (9H,m), 9.0 (1H,broad), 9.8 (1H, broad).

EXAMPLE 2

N-[2-(4-(2-(1-(4-Phenyl)piperazinyl)ethoxy)phenyl)-1-methylethyl]-2-hydroxy-2-(3-trifluoromethylphenyl)ethanamine trihydrobromide

This material was prepared in an identical manner to the compound described in Example 1 using 2-hydroxy-2-(3-trifluoromethylphenyl)ethanamine and 1-[4-(2-(1-(4-phenyl)-piperazinyl)ethoxy)phenyl]propan-2-one. N-[2-(4-(2-(1-(4-phenyl)piperazinyl)ethoxy)phenyl)-1-methylethyl]-2-hydroxy-2-(3-trifluoromethylphenyl)ethanamine trihydrobromide, m.p. 134-138$^{o}$ (methanol-ethyl acetate) was isolated as a 63:37 mixture of diastereoisomers.

$^{1}$H-n.m.r. ;

$\delta$ (DMSO-$d_6$ + $D_2O$)

1.2 (3H,d), 2.6-4.0 (15H,m), 4.5 (2H,m), 5.25 (1H,m), 6.7-7.4 (9H,m), 7.6-7.9 (4H,m).

EXAMPLE 3

N-[2-(4-(2-(1-Piperazinyl)ethoxy)phenyl)-1-methylethyl]-2-hydroxy-2-(3-trifluoromethylphenyl)ethanamine trihydrobromide

N-[2-(4-(2-(1-(4-Benzyl)piperazinyl)ethoxy)phenyl)-1-methylethyl]-2-hydroxy-2-(3-trifluoromethylphenyl)ethanamine trihydrochloride (2.14g) was hydrogenated at ambient temperature and pressure in ethanol containing 10% palladium on carbon. After the theoretical amount of hydrogen had been taken up the mixture was filtered through diatomaceous earth and the solvent evaporated. The residue was partitioned between 2M sodium hydroxide and chloroform, the chloroform layer dried and evaporated. The residual oil was taken up in ether and treated with ethereal hydrogen bromide to give N-[2-(4-(2-(1-piperazinyl)ethoxy)phenyl)-1-methylethyl]-2-hydroxy-2-(3-trifluoromethylphenyl)ethanamine trihydrobromide, m.p. 174-180° (methanol-ethyl acetate) as a 50:50 mixture of diastereoisomers.

$^1$H-n.m.r. ;

δ (DMSO-$d_6$)

1.1 (3H,d), 2.5-3.8 (15H, m, + 1 H disappears with $D_2O$), 4.4 (2H,m), 5.3 (1H,m), 7.0 (2H,d), 7.2 (2H,d), 7.6-7.8 (4H,m), [9.0(1H) + 9.5-10.5(3H), all disappear with $D_2O$].

## EXAMPLE 4

N-[2-(4-(2-(1-(4-Phenyl)piperazinyl)ethoxy)phenyl)-1-methylethyl]-2-hydroxy-2-(3-chlorophenyl)ethanamine trihydrochloride

This material was prepared in an identical manner to the compound described in Example 1 using 2-(3-chlorophenyl)-2-hydroxyethanamine and 1-[4-(2-(1-(4-phenyl)piperazinyl)ethoxy)phenyl]propan-2-one. N-[2-(4-(2-(1-(4-Phenyl)-piperazinyl)ethoxy)phenyl)-1-methylethyl]-2-hydroxy-2-(3-chlorophenyl)ethanamine trihydrochloride, m.p. 163-182$^{o}$C (methanol-ethyl acetate) was isolated as a 59:41 mixture of diastereoisomers.
$^{1}$H-n.m.r. ;
$\delta$ (DMSO-$d_6$)

1.1(3H,d), 2.6-4.0 (16H,m, 1H disappears with $D_2O$), 4.5 (2H,m), 4.95 (1H,m), 6.7-7.5 (13H,m), [9.0 (1H, broad) + 9.8 (1H, broad) + 11.8 (1H, broad) all disappear with $D_2O$].

EXAMPLE 5

N-[2-(4-(2-(1-(4-Phenyl)piperazinyl)ethoxy)phenyl)-1-methylethyl]-2-hydroxy-2-phenylethanamine trihydrochloride

This material was prepared in an identical manner to the compound described in Example 1 using 2-hydroxy-2-phenylethanamine and 1-[4-(2-(1-(4-phenyl)piperazinyl)ethoxy)phenyl]propan-2-one. N-[2-(4-(2-(1-(4-Phenyl)piperazinyl)ethoxy)phenyl)-1-methylethyl]-2-hydroxy-2-phenylethanamine trihydrochloride, m.p. 131-138°C (methanol-ethyl acetate) was isolated as a 50:50 mixture of diastereoisomers.

$^1$H-n.m.r. ;

δ(DMSO-$d_6$)

1.1 (3H,d), 2.6-4.0 (16H,1H disappears with $D_2O$), 4.45 (2H,t), 5.1 (1H,m), 6.8-7.5 (14H,m), [8.9 (1H, broad) + 9.9 (1H, broad) + 11.9 (1H, broad) all disappear with $D_2O$].

EXAMPLE 6

N-[2-(4-(2-(1-(4-Phenyl)piperazinyl)ethoxy)phenyl)-1-methylethyl]-2-hydroxy-2-(2-fluorophenyl)ethanamine trihydrochloride

This material was prepared in an identical manner to the compound described in Example 1 using 2-(2-fluorophenyl)-2-hydroxyethanamine and 1-[4-(2-(1-(4-phenyl)piperazinyl)ethoxy)phenyl]propan-2-one. N-[2-(4-(2-(1-(4-Phenyl)piperazinyl)ethoxy)phenyl)-1-methylethyl]-2-hydroxy-2-(2-fluorophenyl)ethanamine trihydrochloride, m.p. 227-233°C (methanol-ethylacetate) was isolated as a 56:44 mixture of diastereoisomers.

$^{1}$H-n.m.r. ;

δ(DMSO-$d_6$)

1.15 (3H,d), 2.6-4.0 (15H,m), 4.45 (2H,t), 5.3 (2H,m; 1H disappears with $D_2O$), 6.7-7.75 (13H,m), [8.9 (1H, broad), + 9.8 (1H, broad) + 11.8 (1H, broad), all disappear with $D_2O$].

EXAMPLE 7

N-[2-(4-(2-(1-(4-Phenyl)piperazinyl)ethoxy)phenyl)-1-methylethyl]-2-hydroxy-2-(2-fluoro-3-chlorophenyl)ethanamine trihydrochloride

This material was prepared in an identical manner to the compound described in Example 1 using 2-(2-fluoro-3-chlorophenyl)-2-hydroxyethanamine and 1-[4-(2-(1-(4-phenyl)-piperazinyl)ethoxy)phenyl]propan-2-one. N-[2-(4-(2-(1-(4-Phenyl)piperazinyl)ethoxy)phenyl)-1-methylethyl]-2-hydroxy-2-(2-fluoro-3-chlorophenyl)ethanamine trihydrochloride, m.p. 205-219$^{o}$C (methanol-ethylacetate) was isolated as a 55:45 mixture of diastereoisomers.
$^{1}$H-n.m.r. ;
δ(DMSO-$d_6$)

1.15 (3H,d), 2.6-4.0 (15H,m), 4.45 (2H,t), 5.4 (1H,m), 6.6 (1H,s, disappears with $D_2O$), 6.8-7.7 (12H,m), [9.0 (1H, broad) + 9.8 (1H, broad) + 11.8 (1H, broad), all disappear with $D_2O$].

EXAMPLE 8

N-[2-(4-(2-(1-(4-(3-Chlorophenyl))piperazinyl)ethoxy)-phenyl)-1-methylethyl]-2-hydroxy-2-(3-trifluoromethylphenyl)ethanamine trihydrochloride

This material was prepared in an identical manner to the compound described in Example 1 using 2-hydroxy-2-(3-trifluoromethylphenyl)ethanamine and 1-[4-(2-(1-(4-(3-chlorophenyl))piperazinyl)ethoxy)phenyl]propan-2-one. N-[2-(4-(2-(1-(4-(3-Chlorophenyl))piperazinyl)ethoxy)-phenyl)-1-methylethyl]-2-hydroxy-2-(3-trifluoromethylphenyl)ethanamine trihydrochloride, m.p. 169-174°C (methanol-ethyl acetate) was isolated as a 58:42 mixture of diastereoisomers.
$^1$H-n.m.r. ;
δ(DMSO-$d_6$)

1.15 (3H,d), 2.6-4.0 (15H,m), 4.45 (2H,t), 5.25 (1H,m), 6.6-7.8 (13H,m, 1H disappears with $D_2O$) [9.0 (1H, broad), 9.8 (1H, broad), 11.8 (1H, broad) all disappear with $D_2O$].

EXAMPLE 9

N-[2-(4-(2-(1-(4-(3-Trifluoromethylphenyl)) piperazinyl)-ethoxy)phenyl)-1-methylethyl]-2-hydroxy-2-(3-trifluoromethylphenyl)ethanamine trihydrochloride

This material was prepared in an identical manner to the compound described in Example 1 using 2-hydroxy-2-(3-trifluoromethylphenyl)ethanamine and 1-[4-(2-(1-(4-(3-trifluoromethylphenyl))piperazinyl)ethoxy)phenyl]propan-2-one. N-[2-(4-(2-(1-(4-(3-Trifluoromethylphenyl))piperazinyl)-ethoxy)phenyl)-1-methylethyl]-2-hydroxy-2-(3-trifluoromethylphenyl)ethanamine trihydrochloride, m.p. 194-196°C (methanol-ethyl acetate) was isolated as a 37:63 mixture of diastereoisomers.

$^1$H-n.m.r. ;
δ(DMSO-$d_6$)

1.15 (3H,d), 2.6-4.0 (15H,m), 4.4 (2H,t), 5.2 (1H,m), 6.4 (1H, broad, disappears with $D_2O$), 6.8-7.8 (12H,m), [8.9 (1H, broad) + 9.65 (1H, broad), 11.8 (1H, broad) all disappear with $D_2O$].

EXAMPLE 10

N-[2-(4-(2-(1-(4-(4-Chlorophenyl))piperazinyl)ethoxy)-phenyl)-1-methylethyl]-2-hydroxy-2-(3-trifluoromethylphenyl)ethanamine trihydrochloride

This material was prepared in an identical manner to the compound described in Example 1 using 2-hydroxy-2-(3-trifluoromethylphenyl)ethanamine and 1-[4-(2-(1-(4-(4-chlorophenyl))piperazinyl)ethoxy)phenyl]propan-2-one. N-[2-(4-(2-(1-(4-(4-Chlorophenyl))piperazinyl)ethoxy)-phenyl)-1-methylethyl]-2-hydroxy-2-(3-trifluoromethylphenyl)ethanamine trihydrochloride, m.p. 154-162$^{o}$C (methanol-ethyl acetate) was isolated as a 57:43 mixture of diastereoisomers.

$^{1}$H-n.m.r. ;
δ(DMSO-$d_6$)

1.15 (3H,d), 2.6-4.0 (15H + 1H disappears with $D_2O$), 4.4 (2H,t), 5.3 (1H,m), 6.8-7.8 (12H,m), [9.0(1H, broad) + 9.8 (1H, broad) + 11.7 (1H, broad) all disappear with $D_2O$].

EXAMPLE 11

N-[2-(4-(2-(1-(4-(4-Methoxyphenyl))piperazinyl)ethoxy)-phenyl)-1-methylethyl]-2-hydroxy-2-(3-trifluoromethylphenyl)ethanamine trihydrochloride

This material was prepared in an identical manner to the compound described in Example 1 using 2-hydroxy-2-(3-trifluoromethylphenyl)ethanamine and 1-[4-(2-(1-(4-(4-methoxyphenyl))piperazinyl)ethoxy)phenyl]propan-2-one. N-[2-(4-(2-(1-(4-(4-Methoxyphenyl))piperazinyl)ethoxy)-phenyl)-1-methylethyl]-2-hydroxy-2-(3-trifluoromethylphenyl)ethanamine trihydrochloride, m.p. 155-169°C (methanol-ethyl acetate) was isolated as a 57:43 mixture of diastereoisomers.

$^1$H-n.m.r. ;-

δ(DMSO-$d_6$)

1.15 (3H,d), 2.6-4.0 (15H,m), 3.7 (3H,s), 4.45 (2H,t), 5.2 (1H,m), 5.6 (broad, disappears with $D_2O$), 6.8-7.3 (8H,m), 7.5-7.8 (4H,m), [9.0 (1H, broad) + 9.8 (1H, broad), disappears with $D_2O$].

EXAMPLE 12

N-[2-(4-(2-(1-(4-(3-Chlorophenyl))piperazinyl)ethoxy)-phenyl)-1-methylethyl]-2-hydroxy-2-(3-chlorophenyl) ethanamine trihydrochloride

This material was prepared in an identical manner to the compound described in Example 1 using 2-(3-chlorophenyl))-2-hydroxyethanamine and 1-[4-(2-(1-(4-(3-chlorophenyl)-piperazinyl)ethoxy)phenyl]propan-2-one. N-[2-(4-(2-(1-(4-(3-Chlorophenyl))piperazinyl)ethoxy)phenyl)-1-methylethyl]-2-hydroxy-2-(3-chlorophenyl)ethanamine trihydrochloride, m.p. 168-199°C (methanol-ethyl acetate), was isolated as a 21:79 mixture of diastereoisomers.

$^1$H-n.m.r. ;

δ(DMSO-$d_6$)

1.15 (3H,d), 2.6-4.0 (15H,m), 4.4 (2H,t), 5.1 (1H,m) 6.6 (1H, broad, disappears with $D_2O$), 6.7-7.5 (12H,m), [8.9 (1H, broad) + 9.6 (1H, broad) + 11.7 (1H, broad) all disappear with $D_2O$].

EXAMPLE 13

N-[2-(4-(2-(1-(4-(3-Trifluoromethylphenyl))piperazinyl)-ethoxy)phenyl)-1-methylethyl]-2-hydroxy-2-(3-chlorophenyl) ethanamine trihydrochloride

This material was prepared in an identical manner to the compound described in Example 1 using 2-(3-chlorophenyl) -2-hydroxyethanamine and 1-[4-(2-(1-(4-(3-trifluoromethylphenyl)) piperazinyl) ethoxy) phenyl] propan-2-one. N-[2-(4-(2-(1-(4-(3-Trifluoromethylphenyl))piperazinyl)ethoxy) phenyl)-1-methylethyl]-2-hydroxy-2-(3-chlorophenyl)ethan-amine trihydrochloride, m.p. 181-194°C (methanol-ethyl acetate), was isolated as a 51:49 mixture of diastereo-isomers.

$^1$H-n.m.r. ;-
δ(DMSO-$d_6$)

1.15 (3H,d), 2.6-4.0 (15H,m), 4.5 (2H,t), 5.15 (1H,m), 6.9 (broad, disappears with $D_2O$), 6.9-7.6 (12H,m), [8.9 (1H, broad) + 9.7 (1H, broad) disappears with $D_2O$].

EXAMPLE 14

N-[2-(4-(2-(1-(4-Methyl)piperazinyl)ethoxy)phenyl)-1-methylethyl]-2-hydroxy-2-(3-trifluoromethylphenyl) ethanamine trihydrochloride, monohydrate

This material was prepared in an identical manner to the compound described in Example 1 using 2-hydroxy-2-(3-trifluoromethylphenyl)ethanamine and 1-[4-(2-(1-(4-methyl)piperazinyl)ethoxy)phenyl]propan-2-one. N-[2-(4-(2-(1-(4-Methyl)piperazinyl)ethoxy)phenyl)-1-methylethyl]-2-hydroxy-2-(3-trifluoromethylphenyl)ethanamine trihydrochloride, monohydrate, m.p. 160-172°C, (methanol-ethyl acetate) was isolated as a 53:47 mixture of diastereoisomers.

$^1H$-n.m.r. ;-

δ(DMSO-$d_6$)

1.15 (3H,d), 2.6-4.0 (15H,m), 2.85 (3H,s), 2.5-5.5 (3H, broad, disappears with $D_2O$), 4.4 (2H,t), 5.2 (1H,m), 6.9 (2H,d), 7.2 (2H,d), 7.6-7.9 (4H,m), [9.0 (1H, broad) + 9.8 (1H, broad) + 12.0 (1H, broad) all disappear with $D_2O$].

EXAMPLE 15

N-[2-(4-(2-(1-(4-Phenyl)piperazinyl)ethoxy)phenyl)-1-methylethyl]-2-methoxy-2-(3-chlorophenyl)ethanamine trihydrochloride

This material was prepared in an identical manner to the compound described in Example 1 using 2-(3-chlorophenyl)-2-methoxyethanamine and 1-[4-(2-(1-(4-phenyl)piperazinyl)-ethoxy)phenyl]propan-2-one. N-[2-(4-(2-(1-(4-Phenyl)-piperazinyl)ethoxy)phenyl)-1-methylethyl]-2-methoxy-2-(3-chlorophenyl)ethanamine trihydrochloride, m.p. 207-219°C (methanol-ethyl acetate) was isolated as a 44:56 mixture of diastereoisomers.

$^1$H-n.m.r. ;-

δ(DMSO-$d_6$)

1.1 (3H,d), 2.6-4.0 (15H,m), 3.2 (3H,s), 4.5 (2H,t), 4.7 (1H,m), 6.7-7.6 (13H,m), [9.0 (1H, broad) + 9.8 (1H, broad) + 11.8 (1H, broad) all disappear with $D_2O$].

EXAMPLE 16

1-[4-(2-(1-(4-Benzyl)piperazinyl)ethoxy)phenyl]propan-2-one

A mixture of 1-benzylpiperazine (6.2g) and 1-[4-(2-hydroxy-ethoxy)phenyl]propan-2-one, ethylene ketal, 4-toluene sulphonate ester (8.0g) was stirred in dimethyl sulphoxide for 48h at ambient temperature. The solution was poured into water extracted with ethyl acetate and the combined organic layers washed with water and dried. Evaporation of the solvent gave an oil which was stirred in methanol-2M hydrochloric acid for 3h. The solution was extracted with chloroform, the organic layer dried and evaporated to give 1-[4-(2-(1-(4-benzyl)piperazinyl)ethoxy)phenyl]propan-2-one as an oil.

$^1$H-n.m.r. ;-

δ ($CDCl_3$)

2.1 (3H,s), 2.3-2.5 (8H, broad singlet), 2.82 (2H,t), 3.52 (2H,s), 3.68 (2H,s), 4.15 (2H,t), 6.85 (2H,d), 7.15 (2H,d), 7.32 (5H,s).

0102213

EXAMPLE 17

1-[4-(2-(1-(4-Phenyl)piperazinyl)ethoxy)phenyl]propan-2-one

This material was prepared in an identical manner to the compound described in Example 16 using 1-phenylpiperazine (2.43g) and 1-[4-(2-hydroxyethoxy)phenyl]propan-2-one, ethylene ketal, 4-toluenesulphonate ester (3.45g). The crúde oil obtained from deketalisation was chromatographed on Kieselgel 60. Elution with chloroform gave 1-[4-(2-(1-(4-phenyl)piperazinyl)ethoxy)phenyl]propan-2-one as an oil, solidifying on standing.
$^{1}$H-n.m.r. ;-
δ ($CDCl_3$)

2.1 (3H,s), 2.5-3.3 (10H,m), 3.55 (2H, s), 4.05 (2H,t), 6.7-7.35 (9H,m).

EXAMPLE 18

1-[4-(2-(1-(4-Methyl)piperazinyl)ethoxy)phenyl]propan-2-one

This material was prepared in an identical manner to the compound described in Example 17 using 1-methylpiperazine and 1-[4-(2-hydroxyethoxy)phenyl]propan-2-one, ethylene ketal, 4-toluene sulphonate ester.

$^1$H-n.m.r. ;

δ($CDCl_3$)

2.1 (3H,s), 2.3 (3H,s), 2.3-2.8 (8H,m), 2.8 (2H,t), 3.6 (2H,s), 4.1 (2H,t), 6.85 (2H,d), 7.15 (2H,d).

EXAMPLE 19

1-[4-(2-(1-(4-(3-Chlorophenyl))piperazinyl)ethoxy)phenyl] propan-2-one

This material was prepared in an identical manner to the compound described in Example 17 using 1-(3-chlorophenyl) piperazine and 1-[4-(2-hydroxyethoxy)phenyl]propan-2-one, ethylene ketal, 4-toluene sulphonate ester.

$^1$H-n.m.r. ;-

δ($CDCl_3$)

2.1 (3H,s), 2.5-2.8 (4H,m), 2.85 (2H,t), 3.0-3.35 (4H,m) 3.6 (2H,s), 4.1 (2H,t), 6.7-7.3 (8H,m).

0102213

EXAMPLE 20

1-[4-(2-(1-(4-(4-Methoxyphenyl))piperazinyl)ethoxy)phenyl]-propan-2-one

This material was prepared in an identical manner to the compound described in Example 17 using 1-(4-methoxyphenyl)-piperazine and 1-[4-(2-hydroxyethoxy)phenyl]propan-2-one, ethylene ketal, 4-toluene sulphonate ester.
$^1$H-n.m.r. ;-
δ($CDCl_3$)

2.1 (3H,s), 2.5-2.8 (6H,m), 2.8-3.1 (4H,m), 3.5 (2H,s), 3.65 (3H,s), 4.0 (2H,t), 6.7-7.1 (8H,m).

EXAMPLE 21

1-[4-(2-(1-(4-(4-Chlorophenyl))piperazinyl)ethoxy)phenyl]-propan-2-one

This material was prepared in an identical manner to the compound described in Example 17 using 1-(4-chlorophenyl) piperazine and 1-[4-(2-hydroxyethoxy)phenyl]propan-2-one, ethylene ketal, 4-toluene sulphonate ester.

$^1$H-n.m.r. ;-

δ($CDCl_3$)

2.1 (3H,s), 2.6-2.8 (4H,m), 2.81 (2H,t), 3.0-3.3 (4H,m), 3.6 (2H,s), 4.1 (2H,t), 6.7-7.2 (8H,m).

## EXAMPLE 22

1-[4-(2-(1-(4-(3-Trifluoromethylphenyl))piperazinyl)ethoxy)-phenyl]propan-2-one

This material was prepared in an identical manner to the compound described in Example 17 using 1-(3-trifluoromethyl)piperazine and 1-[4-(2-hydroxyethoxy)phenyl]propan-2-one, ethylene ketal, 4-toluene sulphonate ester.
$^1H$-n.m.r. ;-
$\delta(CDCl_3)$

2.1 (3H,s), 2.5-2.8 (4H,m), 2.8 (2H,t), 3.0-3.4 (4H,m), 3.6 (2H,s), 4.1 (2H,t), 6.7-7.3 (8H,m).

## Demonstration of Effectiveness of Compounds

### 1. Anti-obesity activity

The compounds were administered by oral gavage in water or carboxymethyl-cellulose suspension to genetically obese mice daily for 28 days. At the end of the time the carcass composition was determined. The results obtained were as follows:

| COMPOUND OF EXAMPLE NO. | DOSE mg/kg p.o. | g LIPID/MOUSE TREATED | g LIPID/MOUSE CONTROL |
|---|---|---|---|
| 8 | 13.4 | 18.06 | 21.06 |
| 13 | 13.4 | 16.40 | 20.02 |
| 14 | 11.9 | 18.26 | 20.02 |

(2) Effect on Energy Expenditure

The effect of the compounds on the energy expenditure of mice was demonstrated by means of the following procedure:

Female CFLP mice, each weighing approximately 24 g were given food and water *ad lib* before and during the experiment. The compounds were dissolved in water. When necessary the acid addition salt was prepared by addition of hydrochloric acid to the water. These solutions were administered orally to each of 12 mice. A further 12 mice were dosed orally with water. The mice were placed in boxes through which air was drawn and the oxygen content of the air leaving the boxes was measured. The energy expenditure of the mice was calculated for 21 hours after dosing from the volume of air leaving the boxes and its oxygen content, following the principles described by J.B. de V. Weir, J. Physiol. (London), 109, 1-9 (1949). The food intake of the mice was measured over this same period of 21 hours. The results are expressed as a percentage of the mean food intake or rate of energy expenditure of the mice dosed with water.

| Compounds of Example No. | Dose mg/kg po | Mean Energy Expenditure (0-3h) | Mean Energy Expenditure (0-21h) |
|---|---|---|---|
| 1 | 33 | 156 | 114 |
| 2 | 38.5 | 145 | 127 |
| 3 | 34.7 | 146 | 120* |
| 4 | 30.1 | 141 | 126 |
| 5 | 28.5 | 136 | 112 |
| 6 | 29.4 | 127 | 139 |

-continued overleaf-

| | | | |
|---|---|---|---|
| 7 | 31.1 | 121 | 136 |
| 8 | 33.6 | 134 | 126 |
| 9 | 33.4 | 140 | 131 |
| 10 | 33.6 | 128 | 126 |
| 11 | 33.4 | 142 | 128 |
| 12 | 31.8 | 120 | 113 |
| 13 | 33.4 | 139 | 134 |
| 14 | 29.6 | 129 | 125 |
| 15 | 30.8 | 119 | 102 |

*0-19h

3. Hypoglycaemic activity

Female CFLP mice, weighing approximately 25 g, were fasted for 24 hours prior to the study. The compounds under study were administered orally as an aqueous solution to each of 6 mice. 30 minutes later a blood sample (10 µl) was obtained from the tail for the analysis of blood glucose. Immediately after taking this blood sample, glucose (1g/Kg body weight) was administered subcutaneously to each mouse. 6 mice were given water as a control. Blood samples were then obtained from each mouse at 30 minute intervals for 120 minutes.

Compounds that produced a significant ($P<0.05$) reduction of blood glucose, compared with control mice given water, at any time interval, were considered active. The area under the blood glucose curve over the 2 hour period after the administration of the glucose was calculated for each compound and compared with the value for control animals.

| Compounds of Example No. | Dose (µmol/Kg) | % Reduction in area under Blood Glucose Curve |
|---|---|---|
| 1 | 2.5 | 38 |
| 2 | 2.5 | 64 |
| 3 | 2.5 | 20 |
| 15 | 12.5 | 47 |

4. Cardiac Activity

Sprague-Dawley male rats (300-400 g) are killed and the right and left atria dissected out independently and mounted on a combined tissue holder/ electrode. The tissues are then immersed in a glass bath containing Krebs solution maintained at 32°C. The rate of the spontaneously beating right atrium (via an isometric transducer and a ratemeter) and the tension of the electrically paced left atrium (via an isometric transducer) are recorded on an M19 chart recorder.

After an initial stabilization period, the tissues are exposed to a maximal concentration of isoprenaline ($10^{-7}$M). The tissues are then washed several times until rate and tension return to baseline level. A dose-response curve to the test compound is then carried out.

Responses to each concentration of test compound are expressed as a percentage of the maximal responses to isoprenaline. Results are given in the form of (a) intrinsic activity (i.a.) (maximal effect of test compound with respect to isoprenaline maximum, i.e. isoprenaline intrinsic activity = 1) and (b) $EC_{50}$ (the molar concentration at which the test compound produces 50% of its own maximum response).

| Compound of | Rate | | Tension | |
|---|---|---|---|---|
| Example No. | $EC_{50}$(M) | i.a. | $EC_{50}$(M) | i.a. |
| 1 | - | 0 | | 0 |
| 2 | | | | 0 |
| 5 | $5.4 \times 10^{-7}$ | 0.42 | $1.2 \times 10^{-5}$ | 0.67 |
| 6 | $3.5 \times 10^{-7}$ | 0.44 | $1.4 \times 10^{-5}$ | 0.28 |
| 7 | $1.7 \times 10^{-7}$ | 0.35 | - | 0 |
| 10 | $2 \times 10^{-5}$ | 0.86 | $2.7 \times 10^{-5}$ | 1.00 |
| 13 | $3.5 \times 10^{-8}$ | 0.07 | $4.5 \times 10^{-5}$ | 0.33 |
| 14 | $1.7 \times 10^{-6}$ | 0.04 | - | 0 |

## CLAIMS

1. A compound of formula (I):

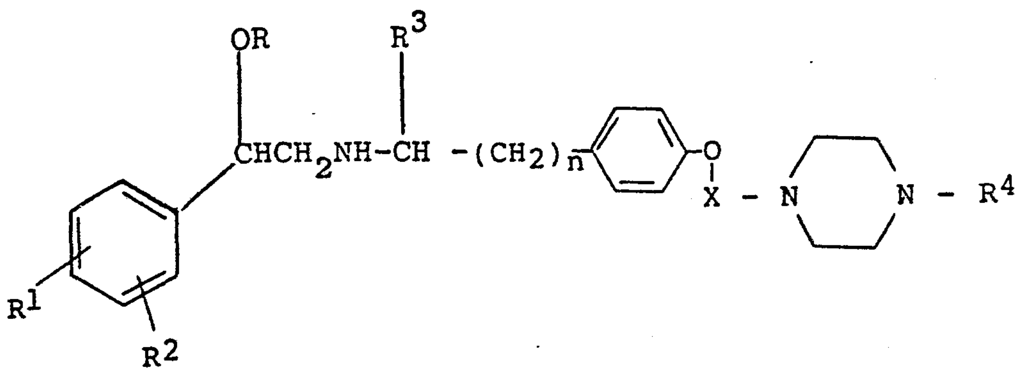

(I)

or a salt thereof,
wherein R is hydrogen or $C_{1-6}$ alkyl,
$R^1$ is hydrogen, or fluorine,
$R^2$ is hydrogen, halogen or trifluoromethyl,
$R^4$ is hydrogen, $C_{1-6}$ alkyl, optionally substituted benzyl, or optionally substituted phenyl,
X is $C_{2-10}$ straight or branched alkylene having at least two carbon atoms interposed between the oxygen and nitrogen atoms.
and n is 1 or 2

2. A compound according to claim 1 wherein R is hydrogen.

3. A compound according to claims 1 or 2, wherein $R^3$ is a methyl group.

4. A compound according to any one of claims 1 to 3, wherein X is ethylene, or n is 1.

5. A compound according to any one of claims 1 to 4, wherein $R^1$ is hydrogen or $R^2$ is a chlorine atom or a trifluoromethyl group in the meta position.

6. A compound according to any one of claims 1 to 5, wherein $R^4$ is an optionally substituted phenyl group.

7. A compound according to claim 1 selected from:

N-[2-(4-(2-(1-(4-benzyl)piperazinyl)ethoxy)phenyl)-1-methylethyl]-2-hydroxy-2-(3-trifluoromethylphenyl)ethanamine trihydrochloride;

N-[2-(4-(2-(1-(4-phenyl)piperazinyl)ethoxy)phenyl)-1-methylethyl]-2-hydroxy-2-(3-trifluoromethylphenyl)ethanamine trihydrobromide;

N-[2-(4-(2-(1-piperazinyl)ethoxy)phenyl)-1-methylethyl]-2-hydroxy-2-(3-trifluoromethylphenyl)ethanamine trihydrobromide;

N-[2-(4-(2-(1-(4-phenyl)piperazinyl)ethoxy)phenyl)-1-methylethyl]-2-hydroxy-2-(3-chlorophenyl)ethanamine trihydrochloride;

N-[2-(4-(2-(1-(4-phenyl)piperazinyl)ethoxy)phenyl)-1-methylethyl]-2-hydroxy-2-phenylethanamine trihydrochloride;

N-[2-(4-(2-(1-(4-phenyl)piperazinyl)ethoxy)phenyl)-1-methylethyl]-2-hydroxy-2-(2-fluorophenyl)-ethanamine trihydrochloride;

N-[2-(4-(2-(1-(4-phenyl)piperazinyl)ethoxy)phenyl)-1-methylethyl]-2-hydroxy-2-(2-fluoro-3-chlorophenyl)ethanamine trihydrochloride;

N-[2-(4-(2-(1-(4-(3-chlorophenyl))piperazinyl)-ethoxy)phenyl)-1-methylethyl]-2-hydroxy-2-(3-trifluoromethylphenyl)ethanamine trihydrochloride;

N-[2-(4-(2-(1-(4-(3-trifluoromethylphenyl))pipera-zinyl)-ethoxy)phenyl)-1-methylethyl]-2-hydroxy-2-(3-trifluoromethylphenyl)ethanamine trihydrochloride;
N-[2-(4-(2-(1-(4-(4-methoxyphenyl))piperazinyl)-ethoxy)phenyl)-1-methylethyl]-2-hydroxy-2-(3-trifluoromethylphenyl)ethanamine trihydrochloride;
N-[2-(4-(2-(1-(4-(3-chlorophenyl))piperazinyl)-ethoxy)phenyl)-1-methylethyl]-2-hydroxy-2-(3-chlorophenyl)ethanamine trihydrochloride;
N-[2-(4-(2-(1-(4-(3-trifluoromethylphenyl))pipera-zinyl)ethoxy)phenyl)-1-methylethyl]-2-hydroxy-2-(3-chlorophenyl)ethanamine trihydrochloride;

N-[2-(4-(2-(1-(4-methyl)piperazinyl)ethoxy)phenyl)-1-methylethyl]-2-hydroxy-2-(3-trifluoromethylphenyl)ethanamine trihydrochloride, monohydrate;
and
N-[2-(4-(2-(1-(4-phenyl)piperazinyl)ethoxy)phenyl)-1-methylethyl]-2-methoxy-2-(3-chlorophenyl)-ethanamine trihydrochloride.

8. A process for preparing a compound of formula (I) or a salt thereof, as claimed in any one of claims 1 to 7, which process comprises reducing a keto-group and/or double bond and/or cleaving a benzyl group(s) of a compound of formula (II):

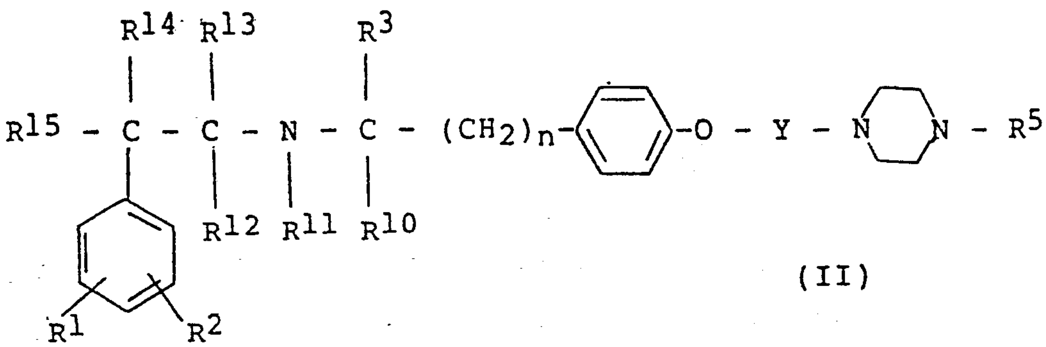

(II)

wherein $R^1$, $R^2$, $R^3$, and n are as defined in relation to formula (I);

$R^5$ is a group $R^4$ as defined in relation to formula (I) or a group reducible to the group $R^4$;

$R^{10}$ is hydrogen or together with $R^{11}$ forms a bond;

$R^{11}$ is hydrogen or benzyl or together with $R^{10}$ or $R^{12}$ forms a bond;

$R^{12}$ is hydrogen or together with $R^{11}$ forms a bond or together with $R^{13}$ forms an oxo-group;

$R^{13}$ is hydrogen or together with $R^{12}$ forms an oxo-group;

$R^{14}$ is hydrogen or together with $R^{15}$ forms an oxo-group;

$R^{15}$ is hydroxyl or $C_{1-6}$ alkoxy, or together with $R^{14}$ forms an oxo-group;

Y is a group X as defined in relation to formula (I) or a group reducible to a group X,

provided that there is no more than one oxo-group represented by any of $R^{12}$ to $R^{15}$;

9. A pharmaceutical composition comprising a compound according to any one of claims 1 to 7 and a pharmaceutically acceptable carrier.

10. A compound according to any one of claims 1 to 7 or a pharmaceutical composition according to claim 9, for use in the treatment of obesity or hyperglycaemia in human or non-human animals.

0102213

Application number

European Patent Office

# EUROPEAN SEARCH REPORT

EP 83304667.5

| DOCUMENTS CONSIDERED TO BE RELEVANT | | | |
|---|---|---|---|
| Category | Citation of document with indication, where appropriate, of relevant passages | Relevant to claim | CLASSIFICATION OF THE APPLICATION (Int. Cl. 3) |
| P,A | EP - A1 - 0 068 669 (BEECHAM GROUP)<br>* Claims 1-3,7,8; page 2, lines 6-11; pages 54, 56 * | 1-4,8-10 | C 07 D 295/08<br>A 61 K 31/495 |
| A | EP - A1 - 0 021 636 (BEECHAM GROUP)<br>* Claims 1-4,11,14,15; pages 36,37 * | 1-4,8-10 | |
| A | EP - A1 - 0 007 206 (ELI LILLY AND COMPANY)<br>* Claims 1,9,13 * | 1-4,8,9 | |
| | | | TECHNICAL FIELDS SEARCHED (Int. Cl. 3) |
| | | | C 07 D 295/00<br>C 07 C 91/00 |

The present search report has been drawn up for all claims

| Place of search | Date of completion of the search | Examiner |
|---|---|---|
| VIENNA | 02-11-1983 | KORBER |

CATEGORY OF CITED DOCUMENTS

X : particularly relevant if taken alone
Y : particularly relevant if combined with another document of the same category
A : technological background
O : non-written disclosure
P : intermediate document
T : theory or principle underlying the invention
E : earlier patent document, but published on, or after the filing date
D : document cited in the application
L : document cited for other reasons
& : member of the same patent family, corresponding document

EPO Form 1503 03.82